# FASCICULE DE BREVET EUROPEEN

(11) **EP 1 212 280 B1**
(45) Date de publication et mention de la délivrance du brevet: **28.07.2004**
(21) Numéro de dépôt: 00962604.5
(22) Date de dépôt: 12.09.2000
(51) Int. Cl.: C07C 51/48, C07C 57/04

(54) **PROCEDE DE PURIFICATION DE L'ACIDE ACRYLIQUE OBTENU PAR OXYDATION DU PROPYLENE ET/OU L'ACROLEINE**
VERFAHREN ZUR REINIGUNG VON DURCH OXIDATION VON PROPYLEN UND/ODER ACROLEIN HERGESTELLTEN ACRYLSÄURE
METHOD FOR PURIFYING ACRYLIC ACID OBTAINED BY OXIDATION OF PROPYLENE AND/OR ACROLEIN

(30) Priorité: 14.09.1999 FR 9911483
(43) Date de publication de la demande: 12.06.2002
(73) Titulaire: Atofina, 92800 Puteaux (FR)
(72) Inventeur: FAUCONET, Michel, F-57730 Valmont (FR); LAURENT, Denis, F-57500 Saint-Avold (FR); STOJANOVIC, Mireille, F-75011 Paris (FR)
(74) Mandataire: Rieux, Michel
(86) Numéro de dépôt international: PCT/FR2000/002505
(87) Numéro de publication internationale: WO 2001/019769

(56) Documents cités:
- EP-A- 0 706 986
- DE-A- 19 627 850
- FR-A- 2 146 386
- FR-A- 2 756 280

## Description

La présente invention porte sur un procédé de purification de l'acide acrylique obtenu par oxydation du propylène et/ou de l'acroléine, par voie catalytique ou par voie redox.

Le principal procédé de synthèse classique de l'acide acrylique utilise une réaction d'oxydation catalytique du propylène à l'aide d'un mélange contenant de l'oxygène. Cette réaction est conduite généralement en phase vapeur, et le plus souvent en deux étapes :
. la première étape réalise l'oxydation sensiblement quantitative du propylène en un mélange riche en acroléine, dans lequel l'acide acrylique est minoritaire ;
. la deuxième étape complète la conversion de l'acroléine en acide acrylique.

Les conditions de réaction de ces deux étapes sont différentes et nécessitent un réglage très précis des paramètres de fonctionnement des réacteurs.

La réaction peut également être conduite dans un seul réacteur, mais, dans ce cas, il est nécessaire de séparer et recycler de grandes quantités d'acroléine à l'étape d'oxydation.

La voie redox a été décrite dans les demandes de brevets français FR-A-2 760 008 et 2 760 009 : la première concerne le premier étage de la fabrication de l'acide acrylique, à savoir la fabrication de l'acroléine par l'oxydation du propylène en phase gazeuse en l'absence d'oxygène moléculaire, en faisant passer le propylène sur une composition solide d'oxydes mixtes particulière, laquelle agit comme système redox et fournit l'oxygène nécessaire à la réaction ; et la seconde concerne le second étage de la fabrication de l'acide acrylique, suivant lequel on fait passer un mélange gazeux d'acroléine et de vapeur d'eau et, le cas échéant, d'un gaz inerte sur une composition solide d'oxydes mixtes particulière, agissant comme système redox et fournissant l'oxygène nécessaire à la réaction.

En règle générale, ces réactions sont conduites dans des réacteurs multitubulaires, les procédés étant dits "à lit fixe" de catalyseur (voie traditionnelle catalytique) ou "à lit fixe" de solide actif (voie nouvelle redox).

On connaît également, pour chaque étage précité (fabrication d'acroléine à partir du , propylène et fabrication d'acide acrylique à partir d'acroléine), un procédé dit "à lit transporté", lequel lit est composé des solides actifs précités enrobés de silice, la réaction étant conduite dans un réacteur de type colonne montante ou "riser".

Les deux procédés d'oxydation énoncés (catalytique et redox) produisent un mélange gazeux réactionnel dont une partie des constituants sont communs. Ce mélange est constitué
- d'une part, de composés incondensables sous des conditions normales de pression et de température : propylène non converti, produits d'oxydation ultimes comme le monoxyde et le dioxyde de carbone, propane ou autres alcanes éventuellement présents en tant qu'impuretés dans le propylène utilisé ; et
- d'autre part, de composés condensables, liquides sous pression atmosphérique et température proche de la température ambiante :
   - acide acrylique ;
   - composés organiques "légers", c'est-à-dire de point d'ébullition inférieur à celui de l'acide acrylique : eau de réaction ; acroléine non convertie ; et composés issus de réactions d'oxydation concurrentes, comme l'acide acétique, le formaldéhyde, etc. ; et
   - composés organiques "lourds", c'est-à-dire de point d'ébullition supérieur à celui de l'acide acrylique : anhydride maléique, furfuraldéhyde, benzaldéhyde, etc.

Outre ces composés communs aux deux procédés, la composition du mélange gazeux issu de la réaction d'oxydation du propylène selon un procédé redox, caractérisé par la séparation des étapes d'oxydation du propylène ou de l'acroléine et de réoxydation du solide actif, est significativement différente de celle d'un procédé catalytique, dans lequel l'oxydation du propylène ou de l'acroléine et la réoxydation du catalyseur sont opérées simultanément. Ainsi, du fait de la séparation des étapes d'oxydation du propylène (en l'absence d'air ou en présence d'une faible quantité d'air) et de régénération du solide actif par l'air, le gaz de réaction d'un procédé redox ne contient pas d'azote, lequel constitue le principal composé inerte incondensable du procédé d'oxydation catalytique classique. Par conséquent, le volume de composés inertes est très réduit en réaction redox.

Par ailleurs, contrairement à un procédé catalytique, la conversion de réactif visée dans le cas d'un procédé redox n'étant pas totale, le mélange gazeux contient une quantité non négligeable de propylène non converti ; et la concentration d'eau et d'acroléine est plus importante dans les gaz issus d'un réacteur redox.

Du fait de ces particularités, la rentabilité du procédé impose de récupérer quantitativement le propylène et l'acroléine non convertis. En revanche, l'eau vapeur doit être partiellement purgée pour éviter son accumulation dans la boucle de recyclage des gaz alimentant le réacteur.

Ces objectifs sont cependant toujours intéressants à atteindre également dans le cas d'une réaction catalytique classique.

La présente invention a pour but de proposer un procédé de purification de l'acide acrylique permettant notamment de récupérer sélectivement et quantitativement, à partir des gaz non condensés de tête de colonne d'absorption, les réactifs non convertis (propylène, acroléine) en vue de les recycler à l'étape de réaction, et de séparer une quantité suffisante d'eau pour éviter son accumulation dans la boucle constituée par les gaz frais introduits dans le réacteur et les gaz recyclés.

Les procédés de purification, décrits dans la littérature, consistent à condenser le mélange gazeux issu de la réaction et à extraire les composés organiques par lavage à contre-courant à l'aide d'eau ou de solvants hydrophobes lourds.

Le brevet français n° 1 558 432 décrit un procédé qui consiste à séparer l'acide acrylique à partir des gaz de réaction d'oxydation du propylène ou de l'acroléine; par absorption à contre-courant à l'aide d'esters d'acide aliphatiques ou aromatiques à points d'ébullition élevés, ou de phosphate de tributyle ou de tricrésyle. A l'issue de cette étape d'absorption, les légers (acroléine, formaldéhyde) sont éliminés en tête d'une première colonne de distillation, et une deuxième colonne de distillation permet d'obtenir en tête une solution aqueuse d'acide acrylique plus concentrée que dans l'art antérieur. Les solvants revendiqués sont relativement polaires et, par conséquent, la solution d'acide acrylique brut obtenue à l'issue de l'étape d'absorption est encore riche en eau. Ceci rend le procédé peu attractif, car la séparation ultérieure de l'eau nécessite encore des opérations coûteuses.

Le brevet français n° 2 002 126 décrit l'utilisation d'un mélange de fractions de point d'ébullition élevé, récupéré en pied des colonnes de purification des esters fabriqués à partir de l'acide acrylique, contenant principalement des maléates, acides polyacryliques, polyacrylates. Le procédé permet de débarrasser en une seule étape, en tête d'une colonne de distillation, la plus grande part des composés de points d'ébullition faibles, tels que l'acroléine, la formaldéhyde, l'eau et l'acide acétique. Toutefois, ce procédé de fabrication d'esters acryliques est mal adapté à la production d'acide acrylique pur, notamment à cause de la présence, dans le mélange d'acide acrylique brut initial, des dérivés d'estérification recyclés à l'étape d'absorption.

Le brevet français n° 2 146 386 décrit un procédé de purification de l'acide acrylique obtenu par absorption à l'aide d'un solvant hydrophobe lourd constitué de diphényle et de diphényléther. Le brevet français n° 2 196 986 décrit un procédé de purification de l'acide acrylique obtenu par absorption à l'aide d'un solvant constitué par un ester d'acide carboxylique de température d'ébullition supérieure à 160°C. Comme exemple de solvant, est cité le phtalate de diéthyle. Le brevet américain n° 5 426 221 décrit un procédé de purification de l'acide acrylique obtenu par absorption à l'aide d'un mélange de solvants hydrophobes lourds constitué de diphényle, diphényléther et phtalate de diméthyle. Ces trois brevets permettent d'obtenir, à l'issue de l'étape d'extraction, une solution anhydre et débarrassée d'une partie substantielle des produits organiques légers qui constituait le mélange gazeux initial (acroléine, formaldéhyde, acide acétique), facilitant ainsi sensiblement la purification ultérieure de l'acide acrylique. Ils ne décrivent pas la récupération d'acroléine et l'élimination d'eau des gaz non absorbés en vue de leur recyclage à l'étape de réaction.

En outre, l'utilisation de solvants relativement polaires du type esters d'acides carboxyliques présente l'inconvénient majeur de favoriser la formation d'impuretés nouvelles, par une réaction secondaire d'hydrolyse conduisant à la dissociation de l'ester en son acide carboxylique et son alcool correspondant, ce dernier pouvant de surcroît réagir avec l'acide acrylique pour générer l'ester acrylique de l'alcool en question. Ainsi, en présence d'eau, le phtalate de diméthyle subit une réaction secondaire d'hydrolyse, favorisée par le niveau thermique de l'étape d'absorption. Cette réaction parasite conduit à la formation d'impuretés nouvelles, comme l'anhydride phtalique et le méthanol qui réagit avec l'acide acrylique pour former l'ester (acrylate de méthyle) correspondant. La formation de ces impuretés complique les étapes ultérieures de purification de l'acide acrylique.

Le brevet français n° 2 287 437 décrit un procédé de récupération d'acide acrylique dans lequel l'acide acrylique contenu dans les gaz de réaction est absorbé à contre-courant, dans une colonne, par un courant liquide envoyé en tête de colonne provenant du recyclage d'une partie du flux extrait en pied de cette colonne. Le courant liquide descendant est à une température inférieure au point de rosée de l'effluent gazeux, sans s'écarter de plus de 15°C de cette température. Ce procédé permet de séparer l'essentiel de l'acroléine en l'éliminant dans les gaz recueillis en tête de colonne, tout en limitant les pertes d'acide acrylique dans ces gaz. Ce procédé est décrit expérimentalement dans le cadre d'une absorption par de l'eau. Son inconvénient majeur est de générer une solution d'acide acrylique brut très diluée dans l'eau (2% d'acide acrylique dans l'eau dans l'Exemple 1), puisque l'essentiel de l'eau est récupéré en pied de colonne. Dans cette hypothèse, l'obtention d'acide acrylique pur à partir de ces solutions nécessiterait un traitement de déshydratation très coûteux. Par ailleurs, le procédé décrit ne pourrait être appliqué dans le cadre d'une absorption par un solvant lourd sans générer des pertes importantes d'acide acrylique en tête.

Le brevet européen n° 706 986 décrit un procédé de récupération d'acide acrylique par absorption à l'aide d'un solvant lourd hydrophobe, dans lequel une section d'absorption à l'eau, située en tête de la colonne d'absorption au solvant lourd, permet de débarrasser le flux gazeux, comprenant les composés non absorbés, de tous les constituants condensables à la température de l'eau froide injectée (l'acroléine fait logiquement partie de ces constituants), et de n'évacuer dans les gaz résiduels que les gaz de dilution inertes (principalement azote) ainsi que les gaz d'oxydation ultime (CO, CO₂). Le procédé ne décrit donc pas les conditions permettant un recyclage de l'acroléine à l'étape de réaction.

De façon surprenante, la Société déposante a découvert que, dans certaines conditions, il est possible d'éliminer sélectivement l'essentiel de l'eau contenue dans les gaz issus de la colonne d'absorption, et avec elle les composés organiques non absorbés présents dans ces gaz, en particulier l'acide acétique, sans condenser les réactifs nobles non convertis (propylène, acroléine) qui peuvent ainsi facilement être recyclés à l'étape de réaction. Le procédé selon l'invention, en permettant de récupérer et de recycler pratiquement quantitativement les réactifs non convertis lors de l'étape réactionnelle, rend très attractif les procédés d'oxydation du propylène et/ou de l'acroléine pour donner l'acide acrylique, en particulier par la voie redox, à conversion non totale, du propylène et de l'acroléine en l'absence d'oxygène ou en présence d'une faible quantité d'oxygène.

Un autre avantage important du procédé selon l'invention découle du fait que, grâce à l'élimination sélective de l'eau, on opère une concentration du gaz résiduaire non condensé en ses constituants récupérables. De ce fait, d'une part le flux recyclé à la réaction est plus concentré en propylène et acroléine, ce qui augmente le rendement de la réaction. D'autre part, cet effet de concentration permet de réduire le débit du flux de purge des gaz résiduaires, nécessaire pour éviter l'accumulation des composés d'oxydation ultimes incondensables (monoxyde et dioxyde de carbone), d'où une réduction de la perte de matières nobles. Enfin, le flux de mélange gazeux résiduaire issu de la colonne d'absorption étant débarrassé de la plus grande partie de son eau, il présente un pouvoir calorifique nettement augmenté qui rend possible sa destruction dans une chaudière productrice de vapeur ou sa réutilisation en tant que combustible, ce qui rend le procédé plus économique en comparaison avec les procédés classiques existants.

La présente invention porte sur un procédé de purification de l'acide acrylique obtenu par oxydation du substrat gazeux propylène et/ou acroléine par voie catalytique ou par voie redox, ledit mélange gazeux issu de ladite, réaction étant principalement constitué par du propylène lorsque le substrat comporte du propylène, des produits d'oxydation ultime, de l'acide acrylique, de l'acroléine, de la vapeur d'eau, de l'acide acétique et des produits lourds de réactions secondaires,
caractérisé par le fait :
- que l'on adresse le mélange gazeux de réaction en pied d'une colonne d'absorption (C1) , laquelle est alimentée en tête et à contre-courant par au moins un solvant lourd d'absorption hydrophobe, pour obtenir :
   - en tête de ladite colonne (C1) , un flux gazeux constitué par le propylène et les produits d'oxydation ultime du mélange, des quantités majeures d'eau et d'acide acétique, et l'acroléine ; et
   - en pied de ladite colonne (C1), un flux constitué par l'acide acrylique, le ou les solvants lourds d'absorption, les produits lourds de réactions secondaires, et des quantités mineures d'acide acétique et d'eau ;
- que l'on adresse le flux gazeux de tête de ladite colonne d'absorption (C1) sur un échangeur de chaleur (C3), où il est mis en contact intime avec un courant liquide descendant, alimenté en tête dudit échangeur de chaleur (C3), et constitué par le recyclage d'une partie du flux de pied de l'échangeur de chaleur (C3) préalablement refroidi, pour obtenir, en tête de l'échangeur de chaleur (C3), un flux gazeux contenant les composés présents dans le flux d'alimentation dudit échangeur de chaleur (C3), excepté la plus grande partie de l'eau et la totalité de l'acide acétique qui sont éliminés dans le flux de pied de l'échangeur de chaleur (C3), et par le fait
qu'on utilise, comme solvant lourd d'absorption hydrophobe, au moins un composé aromatique hydrophobe non hydrolysable, ayant :
- un point d'ébullition sous pression atmosphérique compris entre 260°C et 380°C, de préférence entre 270 et 320°C ;
- une température de cristallisation inférieure à 35°C, de préférence inférieure à 0°C ; et
- une viscosité inférieure à 10 mPa.s dans un interval de température de 30-80°C.

Le gaz de réaction a, d'une manière générale, la composition suivantes (en moles) :
- 3 à 20%, en particulier 7 à 10% d'acide acrylique ;
- 10 à 45%, en particulier 15 à 30% d'eau ;
- 0,1 à 1%, en particulier 0,3 à 0,4% d'acide acétique ;
- 0,1 à 6%, en particulier 1 à 3% d'acroléine ; et
- 40 à 80%, en particulier 50 à 70% d'incondensables.

Le ou les composés aromatiques hydrophobes sont notamment choisis parmi ceux représentés par les formules générales (I) ou (II) : 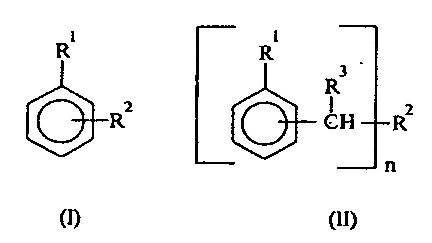 dans lesquelles :
- R¹ représente hydrogène, alkyle en C₁-C₄ ou cycloalkyle;
- R² représente alkyle en C₃-C₈, cycloalkyle, -O-R⁴ (avec R⁴ représentant alkyle en C₃-C₈ ou cycloalkyle), -O-Ph-(R⁵)-R⁶ ou -Ph-(R⁵)-R⁶ (avec R⁵ et R⁶ représentant chacun indépendamment hydrogène ou alkyle en C₁-C₄) (Ph représentant un noyau phénylique) ;
- R³ représente hydrogène ou alkyle en C₁-C₄ ; et
- n vaut 1 ou 2 ;
et ceux représentés par la formule générale (III) : 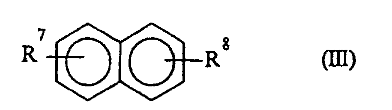 dans laquelle :
- R⁷ représente hydrogène ou alkyle en C₁-C₄ ; et
- R⁸ représente alkyle en C₁-C₄.

De manière préférentielle, on choisit dans cette famille le ditolyléther sous la forme d'un isomère seul ou d'un mélange d'isomères, lequel présente les avantages suivants :
- un seul constituant (pas de problème de séparation par distillation) ;
- séparations facilitées des légers (principalement acide acétique) dans l'étape d'absorption-stripping et des lourds dans les colonnes suivantes ;
- point de figeage très bas (-54°C), ce qui évite tout problème de cristallisation par temps froid.

On peut alimenter la colonne d'absorption (C1) par un ou des solvants purs et/ou par un ou des solvants provenant d'un recyclage à un ou des flux obtenus dans des étapes ultérieures de purification.

Conformément à différents modes de réalisation particuliers de la présente invention :
- on utilise une colonne d'absorption (C1) comportant :
   - dans sa partie inférieure, au moins une section de refroidissement (S1) équipée d'une système de recirculation, à travers un échangeur externe (E1) d'une partie du flux recueilli en partie inférieure de ladite ou desdites sections (S1) pour le renvoyer en tête de ladite ou desdites sections ; et
   - dans sa partie supérieure, une section (S2) d'absorption et de rectification du mélange gazeux ;
- on conduit l'absorption dans la colonne (C1) à la pression atmosphérique ou sous une pression proche de la pression atmosphérique et à une température d'introduction du ou des solvants de 20 à 100°C ;
- on conduit l'absorption dans la colonne (C1) en présence d'au moins un inhibiteur de polymérisation choisi notamment parmi les dérivés phénoliques comme l'hydroquinone et ses dérivés tels que l'éther méthylique de l'hydroquinone, la phénothiazine et ses dérivés, tels que le bleu de méthylène, les quinones, telles que la benzoquinone, les thiocarbamates métalliques, tels que le dibutyldithiocarbamate de cuivre, les composés à groupements nitroso, tels que la N-nitrosophénylhydroxylamine, les amines telles que les dérivés de la paraphénylènediamine. Le ou les inhibiteurs peuvent être introduits avec le solvant d'absorption hydrophobe.

Conformément à un mode de réalisation particulier de la présente invention, on adresse le flux issu de la colonne (C1) à une colonne de distillation (C2) dans laquelle on conduit la distillation pour obtenir :
- en tête, un flux constitué par les impuretés légères que l'on renvoie à la partie inférieure de la colonne d'absorption (C1) ; et
- en pied, un flux constitué par l'acide acrylique en solution dans le ou les solvants d'absorption, une faible proportion d'acide acétique, les produits lourds de réactions secondaires, et le ou les inhibiteurs de polymérisation.

On conduit avantageusement la distillation dans la colonne (C2) sous une pression de 2,66 x 10³ Pa à 3,33 x 10⁴ Pa (soit 20 à 250 mm Hg), à une température de tête de 40 - 90°C et à une température de pied de 60 - 150°C.

L'échangeur de chaleur (C3) est notamment un condenseur à contact direct ou une colonne de condensation partielle.

Par ailleurs, on prévoit avantageusement que le flux gazeux obtenu en tête de l'échangeur de chaleur (C3) soit au moins en partie recyclé à l'étape de réaction.

On peut conduire l'opération dans l'échangeur de chaleur (C3) à la pression atmosphérique ou sous une pression proche de la pression atmosphérique, à une température de tête de 30 - 90°C, de préférence de 50 à 80°C. Le pourcentage d'élimination de l'eau sur l'échangeur de chaleur (C3) est notamment de 20 à 80% en poids, en particulier de 30 à 70% en poids.

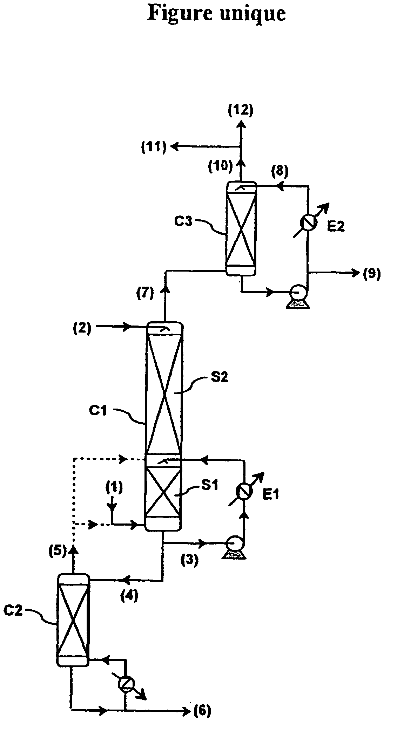

La Figure unique du dessin annexé représente le schéma d'une installation pour la mise en oeuvre du procédé selon la présente invention.

Cette installation comprend une colonne d'absorption C1, une colonne de distillation C2, et une colonne de condensation partielle C3.

### Colonne d'absorption C1

En pied de la colonne d'absorption C1, on envoie un flux 1 constitué par le mélange gazeux de réaction issu de l'oxydation du propylène et de l'acroléine en l'absence d'oxygène, principalement constitué :
- d'une part, de composés incondensables dans les conditions de pression de fonctionnement de la colonne :
   . propylène ;
   . produits d'oxydation ultimes : CO, CO₂ ;
- d'autre part, de composés condensables :
   . acide acrylique ;
   . acroléine ;
   . vapeur d'eau ;
   . acide acétique ;
   . produits de réactions secondaires plus lourds en quantités très faibles.

La colonne d'absorption C1 est alimentée, en tête et à contre-courant, par un flux 2 constitué par un solvant hydrophobe lourd à température d'ébullition supérieure à 200°C sous pression atmosphérique.

De manière préférentielle, la colonne d'absorption C1 comporte :
- dans sa partie inférieure, une ou plusieurs sections de refroidissement S1 équipées d'un système de recirculation, à travers un échangeur externe E1, d'une partie (3) du flux 4 recueilli en partie inférieure de S1, pour le renvoyer en tête de cette section ;
- dans sa partie supérieure, une section S2 dans laquelle s'opère l'absorption et la rectification du mélange.

L'alimentation de solvant (2) est réalisée au-dessus de la section S2. Le solvant introduit peut être un produit pur ou provenir d'un recyclage de flux obtenu dans les étapes ultérieures de purification.

De préférence, la colonne C1 fonctionne sous une pression proche de la pression atmosphérique.

Le flux 4, obtenu en pied de colonne C1, est principalement composé :
- de l'acide acrylique ;
- du solvant ;
- de faibles quantités d'acide acétique et d'eau ; ainsi que
- de stabilisants (inhibiteurs de polymérisation).

### Colonne de distillation C2

De manière avantageuse, on débarrasse le flux 4 de ses impuretés légères en l'envoyant dans la colonne de distillation C2 dans laquelle ces impuretés sont éliminées en tête, avec un peu d'acide acrylique et de solvant.

Le flux 5 ainsi obtenu est renvoyé sur la colonne C1, en un endroit situé dans la partie inférieure de celle-ci, de préférence en tête ou en pied de l'une des sections de refroidissement S1.

Le flux 6, obtenu en pied de colonne C2, est alors constitué principalement par :
- l'acide acrylique en solution dans le solvant ;
- une faible proportion d'acide acétique ;
ainsi que :
- des impuretés lourdes, issues de réactions secondaires, présentes en très faibles quantités dans le flux de gaz de réaction.

De manière avantageuse, la colonne C2 fonctionne sous pression réduite.

### Colonne de condensation partielle C3

Le flux gazeux 7 issu de la colonne C1 contient les composés initialement présents dans le gaz de réaction et non absorbés :
- produits incondensables à la pression de fonctionnement de la colonne : propylène, CO, CO₂ ;
- eau ;
- acroléine ;
- acide acétique.

On envoie ce flux 7 en pied d'une colonne C3 où ce mélange gazeux est mis en contact intime avec un flux 8 de courant liquide descendant alimenté en tête de colonne C3 et constitué par le recyclage d'une partie du flux 9 de pied de colonne C3 préalablement refroidie par un échangeur externe E2.

Le flux gazeux 10 de tête de colonne C3 contient les composés présents dans l'alimentation (flux 7) de la colonne C3, sauf la plus grande partie de l'eau et la totalité de l'acide acétique qui sont éliminés dans le flux 9.

L'essentiel du flux 10 est avantageusement recyclé à l'étape de réaction (flux 11), pour convertir les réactifs nobles qu'il contient. On peut réaliser une faible purge de ce flux (flux 12) pour éviter l'accumulation dans la boucle ainsi constituée de composés incondensables résultant de l'oxydation ultime du propylène : CO, CO₂.

De manière avantageuse, la colonne C3 fonctionne sous une pression proche de la pression atmosphérique.

Les Exemples suivants illustrent la présente invention sans toutefois en limiter la portée. Dans ces Exemples, les pourcentages sont en poids sauf indication contraire.

Le mélange gazeux utilisé dans ces Exemples a une composition fidèle à celle d'un mélange gazeux qui est issu d'un réacteur d'oxydation à lit catalytique transporté fonctionnant dans les conditions de fabrication de l'acide acrylique à partir du propylène. Il est généré par vaporisation complète, dans un flux gazeux d'air, d'un flux liquide composé des principaux constituants du mélange réactionnel réel, à savoir :
- d'acide acrylique ;
- d'acroléine ;
- d'acide acétique ; et
- d'eau.

L'air introduit également dans le vaporisateur remplace, dans des concentrations identiques, le mélange gazeux de composés incondensables, caractéristiques du mélange gazeux réel sortant du réacteur d'oxydation à lit transporté.

On va maintenant décrire l'appareillage utilisé pour la mise en oeuvre des Exemples en faisant référence à la Figure unique du dessin annexé.

### Colonne C1

Le mélange gazeux ainsi généré (1) est envoyé, à une température de 160°C environ, en pied d'une colonne adiabatique C1, de diamètre interne 38 mm, remplie d'une section inférieure de refroidissement (S1), équipée d'un élément de type Sulzer EX, et d'une section supérieure d'absorption/distillation (S2), équipée de dix éléments de garnissage de type Sulzer EX. L'alimentation en gaz réactionnel (1) se fait en bas de la section inférieure de refroidissement (S1).

La colonne C1 est alimentée, en tête de sa section supérieure S2, par un flux 2 constitué d'un mélange des différents isomères du ditolyléther, dans lequel on a préalablement dissous 0,5% d'éther méthylique de l'hydroquinone, en tant qu'inhibiteur de polymérisation. La température d'introduction du solvant d'absorption peut être ajustée au moyen d'un échangeur.

La pression de travail de la colonne C1 est la pression atmosphérique.

### Colonne C2

Le liquide 4 obtenu en pied de la colonne d'absorption C1 est envoyé, au moyen d'une pompe, dans la partie supérieure d'une colonne C2 de diamètre interne 38 mm, équipée de cinq plateaux perforés munis de déversoirs, au niveau de son quatrième plateau compté depuis le bas de la colonne.

La colonne C2 est munie, en pied, d'un bouilleur à thermosiphon, et, en tête, d'un condenseur.

On opère la distillation dans cette colonne C2 sous une pression réduite de 1,87 x 10⁴ Pa (140 mm Hg).

Le flux liquide 6, extrait en pied de cette colonne C2, constitue le mélange brut d'acide acrylique débarrassé des composés légers : incondensables, eau, acroléine, acide acétique en partie.

### Colonne C3

Le flux gazeux non absorbé 7, obtenu en tête de colonne C1, est envoyé en fond d'une colonne C3 de diamètre 38 mm, remplie d'anneaux de verre de diamètre 8 mm sur une hauteur de 40 cm.

Une partie 8 du flux liquide condensé, recueilli en pied de colonne C3, est renvoyée en tête de la même colonne, au moyen d'une pompe, à travers un échangeur E2 qui permet de refroidir le flux liquide 8 à la température désirée.

La proportion de flux de pied de colonne C3 renvoyé en tête de cette colonne est d'environ 10 fois le débit récupéré en pied.

La colonne C3 fonctionne sous pression atmosphérique.

### EXEMPLE 1

Le mélange synthétique de gaz est obtenu par vaporisation totale dans un flux d'air (430 1/h) du mélange en proportion équivalente des composés à l'état liquide

Sa composition est choisie de façon à simuler un gaz réel sortant d'un réacteur d'oxydation du propylène et de l'acroléine par le procédé réalisant l'étape d'oxydation en l'absence d'oxygène sur un catalyseur à lit transporté, dans lequel le taux de conversion du propylène serait de 60%, et celui d'acroléine, de 70%.

Ce mélange est constitué :
- d'une part, des composés condensables :
   . acide acrylique 110,7 g/h (38,8%)
   . eau 139,2 g/h (48,7%)
   . acroléine 31,7 g/h (11,1%)
   . acide acétique 3,9 g/h (1,4%) ;
- d'autre part, d'un flux d'air (430 Nl/h) représentant la somme des composés incondensables présents dans le mélange réactionnel réel.

Ce mélange gazeux 1 est introduit dans la colonne C1 à la température de 152°C. En tête de cette colonne C1, le ditolyléther 2 (mélange des différents isomères + 0,5% d'éther méthylique de l'hydroquinone inhibiteur de polymérisation) est ajouté à un débit de 1341 g/h, et à une température de 50°C. La température en tête de colonne atteint 64,1°C et celle de pied de colonne, 92, 6°C.

La puissance de chauffe appliquée au bouilleur de colonne C2 est ajustée de façon à éliminer toute trace d'eau dans le flux de pied de cette colonne. Le flux 6 d'acide acrylique obtenu en pied de colonne C2 (1497 g/h) contient :
- 7,36% d'acide acrylique ;
- 0,15% d'acide acétique ;
- 0,014% d'acroléine ; et
- moins de 0,01% d'eau.

On récupère 1500 g/h à partir du flux obtenu en pied de colonne C3, pour le renvoyer en tête de cette même colonne après l'avoir refroidi dans l'échangeur. La température des vapeurs incondensées sortant en tête de colonne C3 est de 45°C. Le flux de pied de colonne C3 (123,5 g/h) contient :
- 1,74% d'acide acrylique ;
- 0,89% d'acide acétique ; et
- 0,99% d'acroléine.
Dans ces conditions :
- le taux de récupération d'acide acrylique dans le flux de pied de colonne C2, à partir des gaz de réaction, atteint 99,5% ;
- le taux de récupération de l'acroléine en tête de colonne C3, en vue de son recyclage à l'étape de réaction, est de 94,1% ; et ,
- le taux d'élimination d'eau dans le flux de pied de cette dernière colonne est de 86%.

### EXEMPLE 2

Dans cet exemple, la composition de gaz réactionnel visé à la sortie du vaporiseur est telle qu'elle simule une réaction d'oxydation de propylène et d'acroléine réalisée selon le procédé avec découplage des étapes d'oxydation (en l'absence d'oxygène) et de régénération du catalyseur (en présence d'oxygène), telle que le taux de conversion du propylène, soit de 90% et celui d'acroléine de 90%.

Le mélange gazeux est alors constitué :
- d'une part, des composés condensables :
   . acide acrylique : 116,3 g/h (40,74%) ;
   . eau : 151,2 g/h (53,0%) ;
   . acroléine : 13,3 g/h (4,65%) ;
   . acide acétique : 4,6 g/h (1,64%) ; et
- d'autre part, d'un flux d'air (280 Nl/h) représentant la somme des composés incondensables présents dans le mélange réactionnel réel.

On introduit ce mélange gazeux dans la colonne C1 à la température de 152°C. Il rencontre, à contre-courant, un flux de ditolyléther (1170 g/h), introduit en tête de colonne à une température de 70°C. On mesure en tête de colonne une température de 72,7°C et en pied de colonne 92,7°C.

On applique au bouilleur de colonne C2 une puissance de chauffe telle qu'il n'existe plus d'eau dans le flux de pied de cette colonne. Le flux d'acide acrylique brut obtenu en pied de colonne C2 (1285 g/h) contient :
- 8,86% d'acide acrylique ;
- 0,12% d'acide acétique ;
- 0,003% d'acroléine ; et
- moins de 0,01% d'eau.

On récupère 1500 g/h à partir du flux obtenu en pied de colonne C3, pour le renvoyer en tête de cette même colonne après l'avoir refroidi dans l'échangeur. La température des vapeurs incondensées sortant en tête de colonne C3 est de 44°C Le flux de pied de colonne C3 (133,3 g/h) contient :
- 2,24% d'acide acrylique ;
- 2,06% d'acide acétique ; et
- 0,11% d'acroléine.
Dans ces conditions :
- le taux de récupération d'acide acrylique dans le flux de pied de colonne C2, à partir des gaz de réaction, atteint 97,8% ;
- le taux de récupération de l'acroléine dans le flux de tête de colonne C3, destiné à être recyclé est de 98,1% ; et
- le taux d'élimination d'eau dans le flux de pied de cette dernière colonne est de 84%.

### EXEMPLE 3

L'expérience décrite dans l'Exemple 2 est répétée dans les mêmes conditions, hormis la température à laquelle est refroidi, dans la colonne C3, le mélange gazeux issu de la colonne C2.

Dans le Tableau ci-dessous, on a reporté, pour différentes températures mesurée en tête de colonne C3, les taux de récupération d'acroléine (part d'acroléine récupérée en tête de colonne C3 par rapport à l'acroléine présente initialement dans le gaz de réaction) et d'élimination d'eau (pourcentage d'eau éliminée en pied de colonne C3 par rapport à l'eau initialement présente dans le gaz de réaction).

**TABLEAU**

| | | | | | | |
|---|---|---|---|---|---|---|
| Température tête C3 | 8°C | 28°C | 38°C | 49°C | 56°C | 60°C |
| % de récupération acroléine | 89,9 | 94,5 | 97,2 | 98,1 | 98,8 | 98,6 |
| % d'élimination eau | 97,2 | 93,3 | 91,7 | 84 | 69,5 | 56,8 |

## Revendications

1. Procédé de purification de l'acide acrylique obtenu par oxydation du substrat gazeux propylène et/ou acroléine par voie catalytique ou par voie redox, ledit mélange gazeux (1) issu de ladite réaction étant principalement constitué par du propylène lorsque le substrat comporte du propylène, des produits d'oxydation ultime, de l'acide acrylique, de l'acroléine, de la vapeur d'eau, de l'acide acétique et des produits lourds de réactions secondaires,
**caractérisé par le fait :**
- **que** l'on adresse le mélange gazeux de réaction (1) en pied d'une colonne d'absorption (C1), laquelle est alimentée en tête et à contre-courant par au moins un solvant lourd d'absorption hydrophobe, pour obtenir :
- en tête de ladite colonne (C1) un flux gazeux (7) constitué par le propylène et les produits d'oxydation ultime du mélange (1), des quantités majeures d'eau et d'acide acétique, et l'acroléine ; et
- en pied de ladite colonne (C1), un flux (4) constitué par l'acide acrylique, le ou les solvants lourds d'absorption, les produits lourds de réactions secondaires, et des quantités mineures d'acide acétique et d'eau ;
- **que** l'on adresse le flux gazeux de tête (7) de ladite colonne d'absorption (C1) sur un échangeur de chaleur (C3), où il est mis en contact intime avec un courant liquide descendant (8), alimenté en tête dudit échangeur de chaleur (C3), et constitué par le recyclage d'une partie du flux (9) de pied de l'échangeur de chaleur (C3) préalablement refroidi, pour obtenir, en tête de l'échangeur de chaleur (C3), un flux gazeux (10) contenant les composés présents dans le flux (7) d'alimentation dudit échangeur de chaleur (C3), excepté la plus grande partie de l'eau et la totalité de l'acide acétique qui sont éliminés dans le flux (9) de pied de l'échangeur de chaleur (C3),
et par le fait que l'on utilise, comme solvant lourd d'absorption hydrophobe, au moins un composé aromatique hydrophobe non hydrolysable, ayant :
- un point d'ébullition sous pression atmosphérique compris entre 260°C et 380°C ;
- une température de cristallisation inférieure à 35°C ; et
- une viscosité inférieure à 10 mPa.s dans un intervalle de température de 30-80°C.

2. Procédé selon la revendication 1, **caractérisé par le fait que** l'on choisit un composé aromatique hydrophobe ayant un point d'ébullition compris entre 270°C et 320°C.

3. Procédé selon l'une des revendications 1 et 2, **caractérisé par le fait que** l'on choisit un composé aromatique hydrophobe ayant une température de cristallisation inférieure à 0°C.

4. Procédé selon l'une des revendications 1 à 3, **caractérisé par le fait qu'**on choisit le ou les composés aromatiques hydrophobes parmi ceux représentés par les formules générales (I) ou (II) : 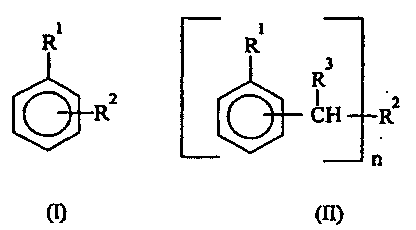 dans lesquelles :
- R¹ représente hydrogène, alkyle en C₁-C₄ ou cycloalkyle;
- R² représente alkyle en C₃-C₈, cycloalkyle, -O-R⁴ (avec R⁴ représentant alkyle en C₃-C₈ ou cycloalkyle), -O-Ph-(R⁵)-R⁶ ou -Ph-(R⁵)-R⁶ (avec R⁵ et R⁶ représentant chacun indépendamment hydrogène ou alkyle en C₁-C₄) (Ph représentant un noyau phénylique) ;
- R³ représente hydrogène ou alkyle en C₁-C₄ ; et
- n vaut 1 ou 2 ;
et ceux représentés par la formule générale (III) 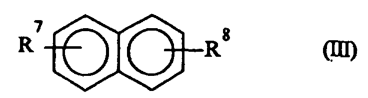 dans laquelle :
- R⁷ représente hydrogène ou alkyle en C₁-C₄ ; et
- R⁸ représente alkyle en-C₁-C₄.

5. Procédé selon l'une des revendications 1 à ,4 **caractérisé par le fait que** l'on alimente la colonne d'absorption (C1) par un ou des solvants purs et/ou par un ou des solvants provenant d'un recyclage d'un ou des flux obtenus dans des étapes ultérieures de purification.

6. Procédé selon l'une des revendications 1 à 5, **caractérisé par le fait que** l'on utilise une colonne d'absorption (C1) comportant :
- dans sa partie inférieure, au moins une section de refroidissement (S1) équipée d'une système de recirculation, à travers un échangeur externe (E1) d'une partie (3) du flux (4) recueilli en partie inférieure de ladite ou desdites sections (S1) pour le renvoyer en tête de ladite ou desdites sections ; et
- dans sa partie supérieure, une section (S2) d'absorption et de rectification du mélange gazeux (1).

7. Procédé selon l'une des revendications 1 à 6, **caractérisé par le fait que** l'on conduit l'absorption dans la colonne (C1) à la pression atmosphérique ou sous une pression proche de la pression atmosphérique et à une température d'introduction du ou des solvants de 20 à 100°C.

8. Procédé selon l'une des revendications 1 à 7, **caractérisé par le fait que** l'on conduit l'absorption dans la colonne (C1) en présence d'au moins un inhibiteur de polymérisation.

9. Procédé selon la revendication 8, **caractérisé par** le fait le ou les inhibiteurs sont choisis parmi les dérivés phénoliques comme l'hydroquinone et ses dérivés tels que l'éther méthylique de l'hydroquinone, la phénothiazine et ses dérivés, tels que le bleu de méthylène, les quinones, telles que la benzoquinone, les thiocarbamates métalliques, tels que le dibutyldithiocarbamate de cuivre, les composés à groupements nitroso, tels que la N-nitrosophénylhydroxylamine, et les amines telles que les dérivés de la paraphénylènediamine.

10. Procédé selon l'une des revendications 1 à 9, **caractérisé par le fait que** l'on adresse le flux (4) issu de la colonne (C1) à une colonne de distillation (C2) dans laquelle on conduit la distillation pour obtenir :
- en tête, un flux (5) constitué par les impuretés légères que l'on renvoie à la partie inférieure de la colonne d'absorption (C1) ; et
- en pied, un flux (6) constitué par l'acide acrylique en solution dans le ou les solvants d'absorption, une faible proportion d'acide acétique, les produits lourds de réactions secondaires, et le ou les inhibiteurs de polymérisation.

11. Procédé selon la revendication 10, **caractérisé par le fait que** l'on conduit la distillation dans la colonne (C2) sous une pression de 2,66 x 10³ Pa à 3,33 x 10⁴ Pa, à une température de tête de 40 - 90°C et à une température de pied de 60 - 150°C.

12. Procédé selon l'une des revendications 1 à 11, **caractérisé par le fait que** l'échangeur de chaleur (C3) est un condenseur à contact direct ou une colonne de condensation partielle.

13. Procédé selon l'une des revendications 1 à 12, **caractérisé par le fait que** le flux (10) est au moins en partie recyclé à l'étape de réaction.

14. Procédé selon l'une des revendications 1 à 13, caractérisé que l'on conduit l'opération dans l'échangeur de chaleur (C3) à la pression atmosphérique ou sous une pression proche de la pression atmosphérique, à une température de tête de 30 - 90°C, le pourcentage d'élimination de l'eau sur l'échangeur de chaleur (C3) étant de 20 à 80% en poids.

## Patentansprüche

1. Verfahren zur Reinigung von durch Oxidation von gasförmigem Propylen- und/oder Acroleinsubstrat auf katalytischem Wege oder auf dem Redoxweg erhaltener Acrylsäure, wobei das bei der Umsetzung anfallende gasförmige Gemisch (1) hauptsächlich aus Propylen, sofern das Substrat Propylen enthält, Oxidationsendprodukten, Acrylsäure, Acrolein, Wasserdampf, Essigsäure und schweren Produkten von Nebenreaktionen besteht,
**dadurch gekennzeichnet, daß**
- man das gasförmige Reaktionsgemisch (1) dem Boden einer Absorptionskolonne (C1) zuführt, welche am Kopf im Gegenstrom mit mindestens einem schweren hydrophoben Absorptionslösungsmittel gespeist wird, wodurch man
- am Kopf der Kolonne (C1) einen gasförmigen Strom (7), der aus Propylen und den Oxidationsendprodukten des Gemischs (1) und größeren Mengen von Wasser und Essigsäure sowie Acrolein besteht, und
- am Boden der Kolonne (C1) einen Strom (4), der aus Acrylsäure, dem oder den schweren Absorptionslösungsmitteln, den schweren Produkten von Nebenreaktionen und kleineren Mengen von Essigsäure und Wasser besteht, erhält;
- man den am Kopf der Absorptionskolonne (C1) anfallenden gasförmigen Strom (7) einem Wärmetauscher (C3) zuführt, in welchem er mit einem nach unten strömenden flüssigen Strom (8), der am Kopf des Wärmetauschers (C3) eingespeist wird und aus der Rückführung eines vorher abgekühlten Teils des Stroms (9) vom Boden des Wärmetauschers (C3) besteht, in innigen Kontakt gebracht wird, wodurch man am Kopf des Wärmetauschers (C3) einen gasförmigen Strom (10) erhält, der die in dem dem Wärmetauscher (C3) zugeführten Strom (7) vorliegenden Verbindungen außer dem größten Teil des Wassers und der gesamten Essigsäure, welche in dem Strom (9) vom Boden des Wärmetauschers (C3) abgetrennt werden, enthält,
und daß man als schweres hydrophobes Absorptionslösungsmittel mindestens eine nicht hydrolysierbare hydrophobe aromatische Verbindung mit
- einem Siedepunkt unter Normaldruck zwischen 260°C und 380°C,
- einer Kristallisationstemperatur von weniger als 35°C und
- einer Viskosität von weniger als 10 mPa.s in einem Temperaturbereich von 30-80°C verwendet.

2. Verfahren nach Anspruch 1, **dadurch gekennzeichnet, daß** man eine hydrophobe aromatische Verbindung mit einem Siedepunkt zwischen 270°C und 320°C wählt.

3. Verfahren nach Anspruch 1 oder 2, **dadurch gekennzeichnet, daß** man eine hydrophobe aromatische Verbindung mit einer Kristallisationstemperatur von weniger als 0°C wählt.

4. Verfahren nach einem der Ansprüche 1 bis 3, **dadurch gekennzeichnet, daß** man die hydrophobe(n) aromatische(n) Verbindung(en) unter denjenigen der allgemeinen Formeln (I) oder (II): 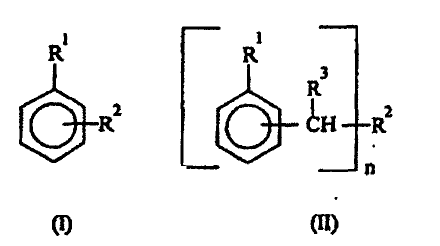 worin:
- R¹ für Wasserstoff, C₁-C₄-Alkyl oder Cycloalkyl steht;
- R² für C₃-C₈-Alkyl, Cycloalkyl, -O-R⁴ (wobei R⁴ C₃-C₈-Alkyl oder Cycloalkyl bedeutet), -O-Ph-(R⁵)-R⁶ oder -Ph- (R⁵) -R⁶ (wobei R⁵ und R⁶ jeweils unabhängig voneinander Wasserstoff oder C₁-C₄-Alkyl bedeuten und Ph einen Phenylring bedeutet) steht;
- R³ für Wasserstoff oder C₁-C₄-Alkyl steht und
- n den Wert 1 oder 2 hat;
und denjenigen der allgemeinen Formel (III) 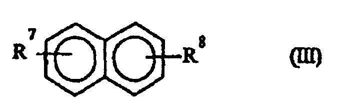 worin:
- R⁷ für Wasserstoff oder C₁-C₄-Alkyl steht und
- R⁸ für C₁-C₄-Alkyl steht;
auswählt.

5. Verfahren nach einem der Ansprüche 1 bis 4, **dadurch gekennzeichnet, daß** man die Absorptionskolonne (C1) mit einem oder mehreren reinen Lösungsmitteln und/oder einem oder mehreren Lösungsmitteln aus der Rückführung eines oder mehrerer, in nachfolgenden Reinigungsstufen erhaltener Ströme speist.

6. Verfahren nach einem der Ansprüche 1 bis 5, **dadurch gekennzeichnet, daß** man eine Absorptionskolonne (C1) verwendet, die
- in ihrem unteren Teil mindestens einen Kühlabschnitt (S1), der mit einem System zum Rezirkulieren eines Teils (3) des im unteren Teil des Abschnitts bzw. der Abschnitte (S1) aufgefangenen Stroms (4) durch einen externen Wärmetauscher (E1) zwecks Rückführung zum Kopf des Abschnitts bzw. der Abschnitte ausgestattet ist, und
- in ihrem oberen Teil einen Abschnitt (S2) zum Absorbieren und Rektifizieren des gasförmigen Gemischs (1)
aufweist.

7. Verfahren nach einem der Ansprüche 1 bis 6, **dadurch gekennzeichnet, daß** man die Absorption in der Kolonne (C1) bei Normaldruck oder einem in der Nähe von Normaldruck liegenden Druck bei einer Zuführungstemperatur für das bzw. die Lösungsmittel von 20 bis 100°C durchführt.

8. Verfahren nach einem der Ansprüche 1 bis 7, **dadurch gekennzeichnet, daß** man die Absorption in der Kolonne (C1) in Gegenwart mindestens eines Polymerisationsinhibitors durchführt.

9. Verfahren nach Anspruch 8, **dadurch gekennzeichnet, daß** man den Inhibitor bzw. die Inhibitoren unter Phenolderivaten, wie Hydrochinon und seinen Derivaten, wie Hydrochinonmethylether, Phenothiazin und seinen Derivaten, wie Methylenblau, Chinonen, wie Benzochinon, Metallthiocarbamaten, wie Kupferdibutyldithiocarbamat, Verbindungen mit Nitrosogruppen, wie N-Nitrosophenylhydroxylamin, und Aminen, wie para-Phenylendiamin-Derivaten, auswählt.

10. Verfahren nach einem der Ansprüche 1 bis 9, **dadurch gekennzeichnet, daß** man den Strom (4) aus der Säule (C1) einer Destillationskolonne (C2) zuführt, in der man durch Destillation
- am Kopf einen aus leichten Verunreinigungen bestehenden Strom (5), der dem unteren Teil der Absorptionskolonne (C1) zugeführt wird, und
- am Boden einen Strom (6), der aus in dem Absorptionslösungsmittel bzw. den Absorptionslösungsmitteln gelöster Acrylsäure, einem geringen Anteil Essigsäure, den schweren Produkten von Nebenreaktionen und dem Polymerisationsinhibitor bzw. den Polymerisationsinhibitoren besteht,
erhält.

11. Verfahren nach Anspruch 10, **dadurch gekennzeichnet, daß** man die Destillation in der Kolonne (C2) unter einem Druck von 2,66 × 10³ Pa bis 3,33 × 10⁴ Pa bei einer Kopftemperatur von 40-90°C und einer Bodentemperatur von 60-150°C durchführt.

12. Verfahren nach einem der Ansprüche 1 bis 11, **dadurch gekennzeichnet, daß** es sich bei dem Wärmetauscher (C3) um einen Direktkontaktkühler oder eine Teilkondensationskolonne handelt.

13. Verfahren nach einem der Ansprüche 1 bis 12, **dadurch gekennzeichnet, daß** man den Strom (10) zumindest teilweise in den Reaktionsschritt zurückführt.

14. Verfahren nach einem der Ansprüche 1 bis 13, **dadurch gekennzeichnet, daß** man den Wärmetauscher (C3) bei Normaldruck oder einem in der Nähe von Normaldruck liegenden Druck und bei einer Kopftemperatur von 30-90°C betreibt, wobei die prozentuale Wasserabtrennung am Wärmetauscher (C3) sich auf 20 bis 80 Gew.-% beläuft.

## Claims

1. Process for the purification of acrylic acid obtained by oxidation of the propylene and/or acrolein gas substrate by the catalytic route or by the redox route, the said gas mixture (1) resulting from the said reaction being composed mainly of propylene, when the substrate comprises propylene, ultimate oxidation products, acrylic acid, acrolein, steam, acetic acid and heavy products from side reactions,
**characterized in that**:
- the reaction gas mixture (1) is sent to the bottom of an absorption column (C1), which column is fed countercurrentwise at the top with at least one heavy hydrophobic absorption solvent, in order to obtain:
- at the top of the said column (C1) a gas flow (7) composed of propylene and the ultimate oxidation products of the mixture (1), major amounts of water and of acetic acid, and acrolein; and
- at the bottom of the said column (C1), a flow (4) composed of acrylic acid, the heavy absorption solvent or solvents, the heavy products from side reactions, and minor amounts of acetic acid and of water;
- the gas flow from the top (7) of the said absorption column (C1) is sent to a heat exchanger (C3), where it is brought into intimate contact with a descending liquid stream (8), fed at the top of the said heat exchanger (C3), composed of the recycling of a portion of the flow (9), cooled beforehand, from the bottom of the heat exchanger (C3), in order to obtain, at the top of the heat exchanger (C3), a gas flow (10) comprising the compounds present in the feed flow (7) of the said heat exchanger (C3), except for most of the water and all the acetic acid, which are removed in the flow (9) from the bottom of the heat exchanger (C3),
and **in that** use is made, as heavy hydrophobic absorption solvent, of at least one nonhydrolysable hydrophobic aromatic compound having:
- a boiling point at atmospheric pressure of between 260°C and 380°C;
- a crystallization temperature of less than 35°C; and
- a viscosity of less than 10 mPa.s in a temperature range of 38-80°C.

2. Process according to Claim 1, **characterized in that** the choice is made of a hydrophobic aromatic compound having a boiling point of between 270°C and 320°C.

3. Process according to either of Claims 1 and 2, **characterized in that** the choice is made of a hydrophobic aromatic compound having a crystallization temperature of less than 0°C.

4. Process according to one of Claims 1 to 3, **characterized in that** the hydrophobic aromatic compound or compounds is/are chosen from those represented by the general formulae (I) or (II): 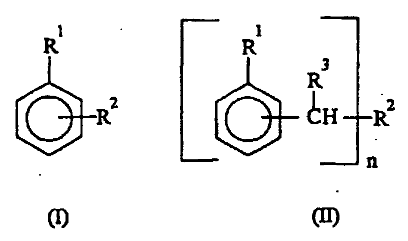 in which:
- R¹ represents hydrogen, cycloalkyl or C₁-C₄ alkyl;
- R² represents cycloalkyl, C₃-C₈ alkyl, -O-R⁴ (with R⁴ representing cycloalkyl or C₃-C₈ alkyl) , -O-Ph-(R⁵)-R⁶ or -Ph-(R⁵)-R⁶ (with R⁵ and R⁶ each independently representing hydrogen or C₁-C₄ alkyl) (Ph representing a phenyl nucleus);
- R³ represents hydrogen or C₁-C₄ alkyl; and
- n has the value 1 or 2;
and those represented by the general formula (III): 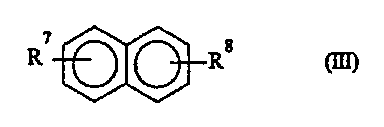 in which:
- R⁷ represents hydrogen or C₁-C₄ alkyl; and
- R⁸ represents C₁-C₄ alkyl.

5. Process according to one of Claims 1 to 4, **characterized in that** the absorption column (C1) is fed with one or more pure solvents and/or with one or more solvents originating from a recycling of one or more flows obtained in subsequent purification stages.

6. Process according to one of Claims 1 to 5, **characterized in that** use is made of an absorption column (C1) comprising:
- in its lower part, at least one cooling section (S1) equipped with a system for recirculating, through an external exchanger (E1), a portion (3) of the flow (4) collected in the lower part of the said section or sections (S1), in order to return it to the top of the said section or sections; and
- in its upper part, a section (S2) for absorbing and rectifying the gas mixture (1).

7. Process according to one of Claims 1 to 6, **characterized in that** the absorption is carried out in the column (C1) at atmospheric pressure or at a pressure close to atmospheric pressure and at a temperature for introducing the solvent or solvents of 20 to 100°C.

8. Process according to one of Claims 1 to 7, **characterized in that** the absorption is carried out in the column (C1) in the presence of at least one polymerization inhibitor.

9. Process according to Claim 8, **characterized in that** the inhibitor or inhibitors are chosen from phenol derivatives, such as hydroquinone and its derivatives, for example hydroquinone methyl ether, phenothiazine and its derivatives, such as methylene blue, quinones, such as benzoquinone, metal thiocarbamates, such as copper dibutyldithiocarbamate, compounds comprising nitroso groups, such as N-nitrosophenylhydroxylamine, and amines, such as para-phenylenediamine derivatives.

10. Process according to one of Claims 1 to 9, **characterized in that** the flow (4) resulting from the column (C1) is sent to a distillation column (C2), in which column distillation is carried out, in order to obtain:
- at the top, a flow (5) composed of the light impurities, which is returned to the lower part of the absorption column (C1); and
- at the bottom, a flow (6) composed of acrylic acid in solution in the absorption solvent or solvents, a low proportion of acetic acid, the heavy products from side reactions, and the polymerization inhibitor or inhibitors.

11. Process according to Claim 10, **characterized in that** the distillation is carried out in the column (C2) at a pressure of 2.66 × 10³ Pa to 3.33 × 10⁴ Pa, at a top temperature of 40-90°C and at a bottom temperature of 60-150°C.

12. Process according to one of Claims 1 to 11, **characterized in that** the heat exchanger (C3) is a direct contact condenser or a partial condensation column.

13. Process according to one of Claims 1 to 12, **characterized in that** the flow (10) is- at least partially recycled to the reaction stage.

14. Process according to one of Claims 1 to 13, **characterized in that** the operation is carried out in the heat exchanger (C3) at atmospheric pressure or at a pressure close to atmospheric pressure and at a top temperature of 30-90°C, the percentage of removal of the water on the heat exchanger (C3) being from 20 to 80% by weight.
